# FASCICULE DE BREVET EUROPEEN

(11) **EP 3 551 048 B1**
(45) Date de publication et mention de la délivrance du brevet: **05.08.2020**
(21) Numéro de dépôt: 17828670.4
(22) Date de dépôt: 07.12.2017
(51) Int. Cl.: A61B 5/00, A61N 1/378

(54) **SYSTÈME IMPLANTABLE**
IMPLANTIERBARES SYSTEM
IMPLANTABLE SYSTEM

(30) Priorité: 07.12.2016 FR 1662058
(43) Date de publication de la demande: 16.10.2019
(73) Titulaire: Université Grenoble Alpes, 38400 Saint-Martin-d'Hères (FR); Centre Hospitalier Universitaire Grenoble Alpes, 38700 La Tronche (FR)
(72) Inventeur: CINQUIN, Philippe, 38330 Saint Nazaire les Eymes (FR); DEFAYE, Pascal, 38000 Grenoble (FR); TUVIGNON, Patrick, 81000 Albi (FR)
(74) Mandataire: Bronchart, Quentin
(86) Numéro de dépôt international: PCT/EP2017/081889
(87) Numéro de publication internationale: WO 2018/104476

(56) Documents cités:
- WO-A1-2011/150032
- WO-A2-2013/080038
- US-A1- 2009 069 803
- US-A1- 2014 058 372
- US-A1- 2015 202 437
- US-A1- 2016 015 988

## Description

La présente invention concerne un système implantable.

Un grand nombre de dispositifs implantables sont utilisés pour surveiller ou stimuler certains organes du corps humain. Par exemple, des dispositifs de stimulation cardiaques (ou « pacemakers ») sont implantés dans de nombreux patients. Ces dispositifs comportent en général une source d'énergie telle qu'une batterie, un ou plusieurs capteurs permettant de surveiller le comportement de l'organe surveillé et/ou un module de stimulation prévu pour exercer une action sur l'organe stimulé.

Cependant, il est nécessaire de recharger ou de remplacer régulièrement les batteries de tels dispositifs implantés. En particulier, dans de nombreux cas ce remplacement est effectué par une opération chirurgicale. Une telle procédure est relativement chère et contraignante pour le patient puisqu'elle a lieu dans un bloc opératoire d'un établissement hospitalier et qu'une anesthésie est nécessaire, ainsi qu'un séjour prolongé dans l'établissement hospitalier afin de surveiller les suites de l'opération. En outre, comme pour toute intervention chirurgicale, il existe des risques que le patient contracte une infection lors de l'opération.

Dans d'autres cas, des dispositifs implantés du type précité sont alimentés depuis l'extérieur par un module de stockage d'énergie qui est porté par le patient à l'extérieur de son corps. Par exemple, il arrive que des dispositifs d'alimentation transmettent de l'énergie par ondes ultrasonores au dispositif de stimulation, à travers la peau et la cage thoracique du patient. Cependant, les ondes ultrasonores traversent mal les os, et une grande précision dans le placement de la source d'ultra-sons est alors nécessaire, dans les cas où le dispositif implanté est situé devant la cage thoracique, afin d'assurer une bonne alimentation du dispositif implanté. En outre, un tel dispositif d'alimentation extérieur au corps du patient est disgracieux.

Il arrive également que des dispositifs implantables soient équipés de connecteurs filaires, permettant une connexion électrique ou un transfert de fluide entre le dispositif implantable et un dispositif extérieur. Par ce biais, un courant électrique d'alimentation ou des données mesurées par les capteurs du dispositif implanté sont échangés avec le dispositif extérieur. Là encore, ces connecteurs débouchant à travers la peau du patient sont disgracieux, et présentent nécessairement des risques sanitaires ainsi que des contraintes importantes pour le patient dans sa vie de tous les jours.

Il existe donc un besoin pour un système implantable qui soit moins contraignant pour le patient. Le document WO-A-2011 /150032 divulgue un système selon le préambule de la revendication 1.

A cet effet, il est proposé un système implantable comprenant un premier dispositif, appelé dispositif de centralisation, propre à être implanté dans une position de fixation à l'intérieur du corps du patient et au moins un deuxième dispositif propre à stimuler un organe du patient lorsque le deuxième dispositif est implanté, dans une position de stimulation, dans le corps du patient, le premier dispositif étant, en outre, configuré pour commander une stimulation de l'organe par le deuxième dispositif, le système implantable étant caractérisé en ce que l'organe est un organe distinct de l'estomac et en ce que, lorsque le premier dispositif est dans la position de fixation, le premier dispositif est accueilli dans l'estomac du patient et fixé à une paroi de l'estomac.

Selon des modes de réalisation, le système implantable comprend l'une ou plusieurs des caractéristiques suivantes, prises isolément ou suivant toutes les combinaisons techniquement possibles :
- le premier dispositif est configuré pour alimenter en énergie le deuxième dispositif ;
- le dispositif de centralisation comporte un émetteur d'ondes acoustiques et le deuxième dispositif comporte un stimulateur propre à stimuler l'organe et un convertisseur d'énergie acoustique en énergie électrique, le convertisseur étant propre à recevoir les ondes acoustiques émises par le dispositif de centralisation et à générer en réponse un courant électrique d'alimentation du stimulateur ;
- l'onde acoustique est une onde ultrasonore ;
- le dispositif de centralisation comporte un émetteur d'ondes électromagnétiques et le deuxième dispositif comporte un stimulateur propre à stimuler l'organe et un convertisseur propre à recevoir les ondes électromagnétiques émises par le dispositif de centralisation et à générer en réponse un courant électrique d'alimentation du stimulateur ;
- l'organe est le cœur du patient ;
- le système implantable comporte au moins deux deuxièmes dispositifs ;
- un deuxième dispositif est prévu pour être implanté dans le ventricule droit et un autre deuxième dispositif est prévu pour être implanté dans le ventricule gauche du cœur du patient ;
- l'organe est un nerf du patient ;
- l'organe est le nerf phrénique du patient ;
- l'organe est le diaphragme du patient ;
- lorsque le dispositif de centralisation est dans la position de fixation, le dispositif de centralisation est accueilli dans la partie supérieure de l'estomac ;
- le dispositif de centralisation ou le deuxième dispositif comporte au moins un capteur propre à mesurer au moins une valeur d'un paramètre de l'organe et le dispositif de centralisation comprend un contrôleur propre à commander la stimulation de l'organe, par le deuxième dispositif, en fonction de la ou les valeurs mesurées ;
- le dispositif de centralisation comprend un contrôleur et une alimentation électrique comportant une réserve d'énergie électrique amovible et un connecteur propre à accueillir la réserve d'énergie électrique, la réserve d'énergie électrique étant propre à alimenter électriquement le contrôleur lorsque la réserve d'énergie électrique est connectée électriquement au connecteur dans une position de connexion et de préférence étant configurée pour être avalée par le patient et pour se déplacer spontanément jusqu'à la position de connexion depuis une position de déconnexion dans laquelle la réserve d'énergie électrique est accueillie dans l'estomac du patient (P) et est déconnectée du connecteur ;
- le dispositif de centralisation comprend un contrôleur et une alimentation électrique propre à générer un courant électrique d'alimentation du contrôleur par réaction d'au moins une espèce chimique présente dans le corps du patient, notamment le glucose ;
- le dispositif de centralisation comprend un contrôleur et une alimentation électrique propre à générer un courant électrique d'alimentation du contrôleur par conversion d'énergie mécanique en énergie électrique.

Des caractéristiques et avantages de l'invention apparaîtront à la lecture de la description qui va suivre, donnée uniquement à titre d'exemple non limitatif et faite en référence aux dessins annexés, sur lesquels :
- la figure 1 est un schéma d'un exemple de système implantable comportant une alimentation électrique,
- la figure 2 est une représentation schématique du dispositif implantable de la figure 1, implanté dans le corps d'un patient, et
- la figure 3 est une représentation schématique de l'alimentation de la figure 1.

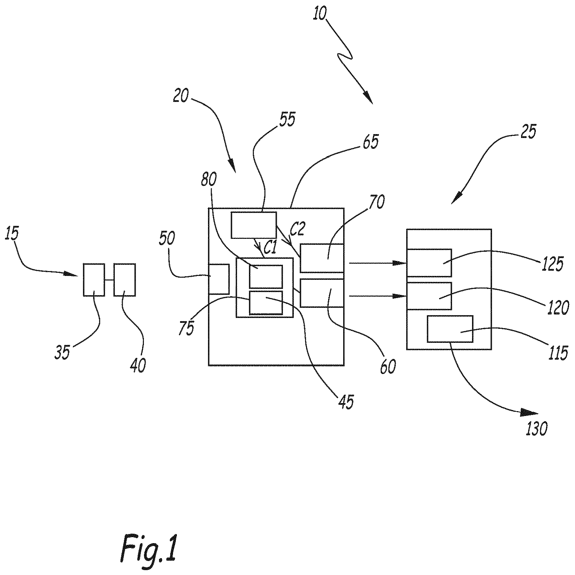

Un premier exemple de système implantable 10 est représenté à la figure 1.

Le système implantable 10 comporte une ancre 15, un premier dispositif 20, appelé dispositif de centralisation, et au moins un deuxième dispositif 25.

Il est entendu par « système implantable » qu'au moins un élément parmi la liste formée de l'ancre 15, du premier dispositif 20 et du deuxième dispositif 25 est prévu pour être implanté dans le corps humain.

En particulier, il est entendu par « implantable » qu'au moins un élément parmi l'ancre 15, le dispositif de centralisation 20 et le deuxième dispositif 25 est prévu pour séjourner dans le corps d'un patient P pendant une durée strictement supérieure à une semaine, de préférence strictement supérieure à un mois, de préférence supérieure ou égale à un an.

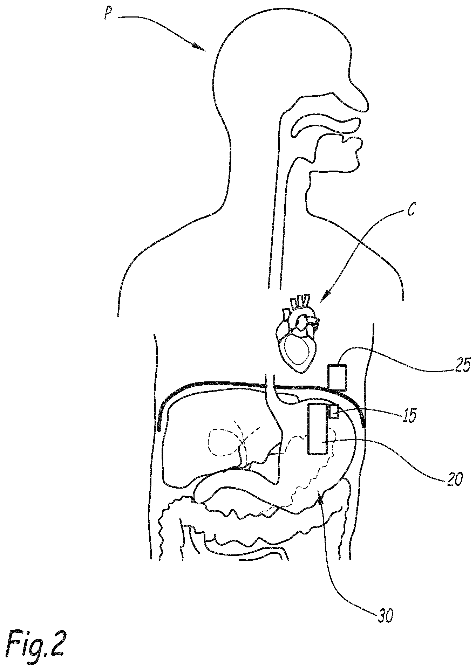

Le système implantable 10 a été représenté schématiquement sur la figure 2 lorsque le système implantable 10 est implanté dans le corps du patient P.

Selon l'exemple de la figure 2, l'ancre 15, le dispositif de centralisation 20 et le deuxième dispositif 25 sont chacun implanté dans le corps du patient P.

L'ancre 15 est propre à être fixée dans une position prédéterminée dans l'estomac 30 du patient P.

Par exemple, l'ancre 15 est configurée pour être fixée dans la partie supérieure de l'estomac 30. En particulier, l'ancre 15 est configurée pour être fixée dans le fundus gastrique de l'estomac 30. Par exemple, l'ancre 15 est prévue pour être fixée le plus près possible de l'angle de Hiss dans le fundus gastrique.

En variante, l'ancre 15 est configurée pour être fixée dans la partie inférieure de l'estomac 30.

L'ancre 15 est configurée pour supporter le dispositif de centralisation 20, de préférence de manière amovible. En particulier, l'ancre 15 et le dispositif de centralisation 20 sont configurés pour être fixés l'un à l'autre, par un dispositif de fixation, et l'ancre 15 est configurée pour maintenir le dispositif de centralisation 20 dans une position de fixation lorsque l'ancre 15 est fixée dans l'estomac 30.

L'ancre 15 comporte une tête 35 et un premier connecteur 40.

La tête 35 est configurée pour ancrer l'ancre 15 dans la position prédéterminée. En particulier, la tête 35 est configurée pour ancrer l'ancre 15 à la paroi de l'estomac 30.

La tête 35 est, par exemple, un clip gastro-intestinal configuré pour enserrer entre deux branches de la tête 35 une portion de la paroi de l'estomac 30.

En variante, la tête 35 est propre à être suturée par un fil à la paroi de l'estomac 30.

Selon une autre variante, la tête 35 est propre à être enfouie à l'intérieur de la muqueuse gastrique après que celle-ci ait été disséquée.

Le premier connecteur 40 est configuré pour fixer le dispositif de centralisation 20 à la tête 35.

Le dispositif de centralisation 20 est configuré pour alimenter en énergie le deuxième dispositif 25.

Le dispositif de centralisation 20 comporte un premier contrôleur 45, un deuxième connecteur 50, une alimentation électrique 55, un premier émetteur/récepteur 60, un boîtier 65 et un transmetteur 70.

Le premier contrôleur 45 est une unité de traitement d'informations. Le premier contrôleur 45 comporte une première mémoire 75 et un premier processeur 80.

En variante, le premier contrôleur 45 est réalisé sous forme de circuit intégré dédié, ou encore de composants logiques programmables.

Le premier processeur 80 est propre à manipuler et/ou transformer des données représentées comme des quantités électroniques ou physiques dans la première mémoire 75 en d'autres données similaires correspondant à des données physiques dans la première mémoire 75, dans des registres ou d'autres types de dispositifs d'affichage, de transmission ou de mémorisation.

Le premier processeur 80 est, en outre, configuré pour échanger des informations avec le premier émetteur/récepteur 60.

Le deuxième connecteur 50 est configuré pour coopérer avec le premier connecteur 40 pour maintenir le dispositif de centralisation 20 dans la position de fixation.

Par exemple, le deuxième connecteur 50 est configuré pour coopérer avec le premier connecteur 40 par encliquetage.

En variante, le deuxième connecteur 50 comporte un aimant configuré pour fixer le deuxième connecteur au premier connecteur. L'aimant est, par exemple, un électroaimant.

Selon une autre variante, le premier connecteur 40 est configuré pour être solidarisé au deuxième connecteur 50 par vissage. En variante, le premier connecteur 40 comporte une ou de préférence deux baïonnettes complémentaires d'orifices de fixation ménagés dans le deuxième connecteur 50.

De préférence, le deuxième connecteur 50 est prévu pour que le dispositif de centralisation 20 soit séparable de l'ancre 15. En particulier, le deuxième connecteur 50 est configuré pour que le dispositif de centralisation 20 soit séparable de l'ancre 15 lorsque l'ancre 15 est fixée dans l'estomac 30 du patient P.

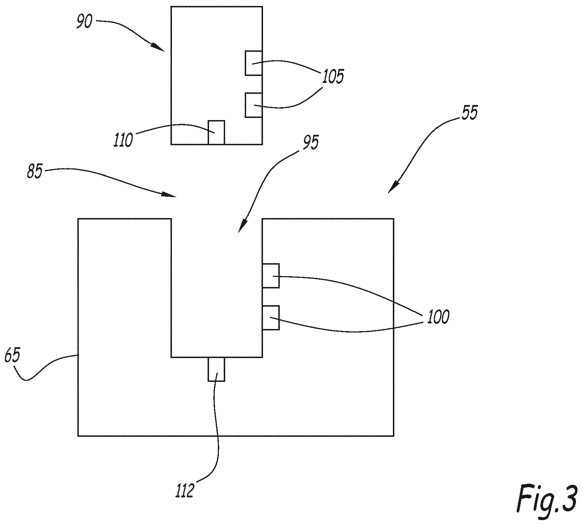

L'alimentation électrique 55 a été représentée sur la figure 3.

L'alimentation électrique 55 est configurée pour alimenter le premier contrôleur 45 avec un premier courant d'alimentation C1.

L'alimentation électrique 55 est, en outre, configurée pour alimenter le transmetteur 70 avec un deuxième courant d'alimentation C2.

L'alimentation électrique 55 comporte un troisième connecteur 85 et une première réserve d'énergie électrique 90.

Le troisième connecteur 85 est configuré pour recevoir de la première réserve d'énergie électrique 90 le premier courant d'alimentation C1 et le deuxième courant d'alimentation C2 et pour alimenter le premier contrôleur 45 et le transmetteur 70 avec, respectivement, le premier courant d'alimentation C1 et le deuxième courant d'alimentation C2.

Le troisième connecteur 85 est configuré pour accueillir la première réserve d'énergie électrique 90. En particulier, le troisième connecteur 85 délimite une cavité 95 configurée pour accueillir au moins partiellement la première réserve d'énergie électrique 90 dans une position de connexion.

Selon l'exemple de la figure 3, la cavité 95 débouche sur l'extérieur du boîtier 65. En particulier, la cavité 95 est configurée pour permettre l'insertion de la première réserve d'énergie électrique 90, depuis l'extérieur du boîtier 65, dans la cavité 95.

Le troisième connecteur 85 comporte, en outre, deux premiers contacts électriques 100, configurés pour être connectés électriquement à la première réserve d'énergie électrique 90 lorsque la première réserve d'énergie électrique 90 est dans la position de connexion. En particulier, les deux premiers contacts électriques 100 débouchent à l'intérieur de la cavité 95.

La première réserve d'énergie électrique 90 est configurée pour stocker de l'énergie électrique. En particulier, la première réserve d'énergie électrique 90 est configurée pour être chargée en énergie électrique à l'extérieur du corps du patient P et pour se décharger lorsque la première réserve d'énergie électrique 90 est dans la position de connexion. Par exemple, la première réserve d'énergie électrique 90 comporte une batterie. En variante, la première réserve d'énergie électrique 90 comporte au moins un capaciteur ou une supercapacité.

La première réserve d'énergie électrique est configurée pour alimenter le premier contrôleur 45 avec le premier courant d'alimentation C1 lorsque la première réserve d'énergie électrique 90 est dans la position de connexion. En outre, la première réserve d'énergie électrique 90 est configurée pour alimenter le transmetteur 70 avec le deuxième courant d'alimentation C2 lorsque la première réserve d'énergie électrique 90 est dans la position de connexion.

Selon l'exemple de la figure 3, la première réserve d'énergie électrique 90 comporte deux deuxièmes contacts électriques 105 complémentaires des premiers contacts électriques 100.

La première réserve d'énergie électrique 90 peut être prévue pour être avalée par le patient P.

Selon une variante, la première réserve d'énergie 90 est propre à être remplacée par endoscopie.

En particulier, la première réserve d'énergie électrique 90 présente un volume strictement inférieur à 6 millilitres (ml).

La première réserve d'énergie électrique 90 présente, en outre, trois dimensions mesurées chacune selon une direction respective, chaque direction étant perpendiculaire aux deux autres directions, et chaque dimension est strictement inférieure à 5 centimètres (cm).

La première réserve d'énergie électrique 90 est mobile entre la position de connexion et une position de déconnexion. Lorsque la première réserve d'énergie électrique 90 est dans la position de déconnexion, la première réserve d'énergie électrique 90 est accueillie dans l'estomac 30 du patient P mais n'est pas connectée électriquement au troisième connecteur 85. Par exemple, lorsque la première réserve d'énergie électrique 90 est dans la position de déconnexion, la première réserve d'énergie électrique est totalement extraite de la cavité 95.

La première réserve d'énergie électrique 90 est configurée pour se déplacer spontanément depuis la position de déconnexion vers la position de connexion. Par exemple, la première réserve d'énergie électrique 90 comporte des attracteurs 110.

Les attracteurs 110 sont configurés pour exercer sur la première réserve d'énergie électrique 90, lorsque la première réserve d'énergie électrique 90 est dans la position de déconnexion, une force tendant à déplacer la première réserve d'énergie électrique 90 depuis la position de déconnexion vers la position de connexion.

En outre, les attracteurs 110 sont configurés pour maintenir la première réserve d'énergie électrique 90 dans la position de connexion.

Les attracteurs 110 comportent, par exemple, un premier aimant propre à coopérer avec un deuxième aimant 112 du troisième connecteur 85. En variante, le premier aimant est propre à coopérer avec une portion ferromagnétique du troisième connecteur 85. Le premier aimant et le deuxième aimant 112 sont, par exemple, des électro-aimants.

Le premier émetteur/récepteur 60 est configuré pour échanger des informations avec le deuxième dispositif 25. Le premier émetteur/récepteur 60 forme ainsi des moyens de communication avec le deuxième dispositif 25.

Le premier émetteur/récepteur 60 est, par exemple, un module de communication radiofréquence. Il est entendu par « module de communication radiofréquence » que le premier émetteur/récepteur 60 est configuré pour communiquer avec le deuxième dispositif 25 via un signal comportant au moins une onde électromagnétique radiofréquence. Les ondes électromagnétiques radiofréquences sont les ondes électromagnétiques présentant une fréquence comprise entre 3 kilohertz et 3 gigahertz.

Selon un mode de réalisation, le premier émetteur/récepteur 60 est propre à échanger des informations avec le deuxième dispositif 25 selon un protocole Bluetooth Low Energy. Le protocole Bluetooth Low Energy est un protocole basé sur un standard du « Bluetooth special interest group » et fonctionnant dans la gamme comprise entre 2400 mégahertz (MHz) et 2483.5 MHz.

En variante, des modes de transmission d'information dans les gammes 402 - 405 mégahertz (MHz) (Medical Implant Communication Service) ou 2360 - 2390 MHz (Medical Body Area Networks) peuvent être utilisés.

Le boîtier 65 est configuré pour isoler le premier contrôleur 45 de l'extérieur du boîtier 65. Par exemple, le boîtier 65 délimite une chambre recueillant au moins le premier contrôleur 45, le premier émetteur/récepteur 60 et le transmetteur 70.

Le transmetteur 70 est configuré pour transmettre de l'énergie au deuxième dispositif 25.

Par exemple, le transmetteur 70 comporte un émetteur/récepteur d'ondes acoustiques configuré pour émettre une onde acoustique et pour diriger l'onde acoustique sur le deuxième dispositif 25.

En variante, le transmetteur 70 est configuré pour émettre une onde électromagnétique. L'onde électromagnétique présente, par exemple, une haute fréquence telle qu'une fréquence de l'ordre de 13,56 Mégahertz (MHz). Par exemple, le transmetteur 70 comporte une bobine configurée pour émettre l'onde électromagnétique.

En variante, le transmetteur 70 est relié au deuxième dispositif 25 par un conducteur électrique et est configuré pour transmettre un courant électrique au deuxième dispositif 25 par liaison filaire.

Le deuxième dispositif 25 est propre à stimuler un organe C du patient P.

L'organe C est distinct de l'estomac 30 du patient P.

Le deuxième dispositif 25 est configuré pour être implanté dans le corps du patient P dans une position de stimulation. Le deuxième dispositif 25 est alors configuré pour stimuler l'organe C lorsque le deuxième dispositif 25 est dans la position de stimulation.

Lorsque le deuxième dispositif 25 est dans la position de stimulation, le deuxième dispositif 25 est situé en dehors de l'estomac 30.

Il est entendu par « stimuler » que le deuxième dispositif 25 est propre à exercer une action sur l'organe C et à provoquer en réponse une action de l'organe C. Une contraction musculaire est un exemple d'action d'un organe C. La transmission d'un signal nerveux est un autre exemple d'action.

L'organe C est le cœur du patient P.

Dans un mode de réalisation, le deuxième dispositif 25 peut être implanté dans l'endocarde du cœur du patient P, par exemple dans le ventricule droit, lorsque le deuxième dispositif est dans sa position de stimulation.

Le deuxième dispositif 25 comporte un stimulateur 115, un deuxième émetteur/récepteur 120, et un convertisseur 125.

Le stimulateur 115 est configuré pour stimuler l'organe C. En particulier, le stimulateur 115 est configuré pour provoquer une contraction du cœur du patient P.

Le stimulateur 115 comporte, par exemple, une électrode 130 connectant électriquement le deuxième dispositif 25 à un emplacement prédéterminé de l'organe C du patient P.

Le deuxième émetteur/récepteur 120 est configuré pour échanger des informations avec le premier émetteur/récepteur 60.

Le convertisseur 125 est configuré pour recevoir de l'énergie du transmetteur 70 et pour convertir l'énergie reçue en énergie électrique.

En particulier, le convertisseur 125 est configuré pour alimenter le stimulateur 115 avec un troisième courant d'alimentation C3.

Par exemple, le convertisseur 125 est configuré pour recevoir l'onde acoustique émise par le transmetteur 70 et pour générer en réponse le troisième courant d'alimentation C3.

Le convertisseur 125 comporte, par exemple, des éléments piézo-électriques propres à convertir une force en tension électrique. En particulier les éléments piézo-électriques sont propres à convertir l'onde acoustique émise par le transmetteur 70 en tension électrique.

En variante, le convertisseur 125 est configuré pour recevoir une onde électromagnétique émise par le transmetteur 70 et pour générer en réponse le troisième courant d'alimentation C3. Le convertisseur 125 comporte, par exemple, une bobine propre à résonner à la fréquence de l'onde électromagnétique émise par le transmetteur 70.

Le fonctionnement du système implantable 10 va maintenant être décrit.

Au cours d'une première étape préalable à l'implantation de l'ancre 15, du dispositif de centralisation 20 et du deuxième dispositif 25 dans le corps du patient P, la première réserve d'énergie électrique 90 est chargée en énergie électrique. La première réserve d'énergie électrique 90 génère donc le premier courant d'alimentation C1 à destination du premier contrôleur 45.

Au cours d'une deuxième étape, l'ancre 15, le dispositif de centralisation 20 et le deuxième dispositif 25 sont implantés dans le corps du patient P.

Au cours d'une troisième étape, un message d'activation est transmis, par un dispositif externe, au dispositif de centralisation 20. En particulier, le message d'activation est transmis par communication radiofréquence. Le message d'activation informe le premier contrôleur 45 que le système implantable 10 a bien été implanté dans le corps du patient P.

Au cours d'une quatrième étape postérieure à la troisième étape, le premier contrôleur 45 commande l'alimentation du deuxième dispositif 25 par le transmetteur 70. Par exemple, le premier contrôleur 45 commande la fermeture d'un interrupteur connectant électriquement le troisième connecteur 85 au transmetteur 70. Le troisième connecteur 85 transmet alors le deuxième courant d'alimentation C2 au transmetteur 70.

Lors de la quatrième étape, le transmetteur 70 émet alors une onde acoustique et dirige l'onde acoustique vers le deuxième dispositif 25.

L'onde acoustique est, par exemple, une onde ultrasonore. Les ondes ultrasonores sont les ondes acoustiques présentant une fréquence comprise entre 20 kilohertz et 100 mégahertz.

Au cours d'une cinquième étape, le convertisseur 125 reçoit l'onde émise par le transmetteur 70. Le convertisseur 125 convertit au moins une partie de l'énergie de l'onde reçue en énergie électrique. Le convertisseur 125 génère alors le troisième courant électrique C3 à partir de l'onde reçue et alimente le stimulateur 115 avec le troisième courant électrique C3.

Au cours d'une sixième étape, le troisième courant électrique C3 est transmis jusqu'au cœur du patient P par l'électrode 130. Le cœur du patient se contracte alors en réponse au troisième courant électrique C3. Par exemple, le troisième courant électrique C3 est propre à corriger un trouble du rythme cardiaque tel qu'une fibrillation.

Au cours d'une septième étape, le premier contrôleur 45 commande l'interruption du deuxième courant d'alimentation C2. Le deuxième dispositif 25 n'est donc plus alimenté en énergie, et le troisième courant électrique C3 n'est donc plus transmis au cœur du patient P. La stimulation de la contraction du cœur C se termine donc.

Les quatrième, cinquième, sixième et septième étapes sont, par exemple, répétées successivement dans cet ordre avec une période temporelle prédéterminée. La période temporelle est, typiquement, une période correspondant au nombre de contractions par minute souhaité, par exemple environ 70 contractions par minute.

Grâce à l'invention, l'alimentation électrique du deuxième dispositif 25 est assurée depuis le dispositif de centralisation 20. Le deuxième dispositif 25 est alors un dispositif passif, puisqu'il n'est activé que par la transmission d'énergie depuis le dispositif de centralisation 20. Le deuxième dispositif 25 a pour seule fonction la conversion de l'onde émise par le dispositif de centralisation 20 en un courant électrique transmis au cœur C.

Le deuxième dispositif 25 est alors simple à réaliser. De plus, le volume du deuxième dispositif 25 est faible, puisqu'il ne contient pas de réserve d'énergie électrique. L'implantation du deuxième dispositif 25 est donc rendue plus aisée, et est possible dans un plus grand nombre d'emplacements.

Du fait que le dispositif de centralisation 20 est dans l'estomac, le remplacement de la première réserve d'énergie électrique 90 est aisé et peut, par exemple, être effectué par voie endoscopique depuis l'œsophage, de manière simple et rapide. En outre, le remplacement de la première réserve d'énergie électrique 90 pose peu de risques d'infection puisque aucune incision n'est réalisée.

L'utilisation des attracteurs 110 rend encore plus simple la mise en place de la première réserve d'énergie électrique 90, même sans endoscopie, puisqu'il suffit alors que le patient P avale la première réserve d'énergie électrique 90.

En outre, le système implantable 10 ne suppose pas que le patient P porte en permanence des moyens de stockage d'énergie électrique à l'extérieur de son corps, ni que des conducteurs électriques disgracieux débouchent hors du corps du patient P. Le système implantable 10 est donc peu contraignant pour le patient.

Le positionnement du dispositif de centralisation dans l'estomac 30 du patient P permet d'interagir efficacement avec le deuxième dispositif 25, et ce pour une grande variété d'organes C et donc une grande variété d'emplacements du deuxième dispositif 25.

En effet, bien que le premier exemple ait été décrit dans le cas d'une stimulation cardiaque, il est à noter que l'invention est susceptible d'être appliquée à un grand nombre d'organes C distincts.

Selon une variante, l'organe C est un nerf du patient P.

Par exemple, l'organe C est le nerf phrénique du patient P. Par exemple, le deuxième dispositif 25 est configuré pour stimuler électriquement le nerf phrénique.

En variante, l'organe C est un nerf dont la stimulation permet de supprimer un signal nerveux d'une douleur du patient P.

Selon une autre variante, l'organe C est un muscle du patient P différent du cœur. Par exemple, l'organe C est le diaphragme du patient P.

Selon une autre variante, le système implantable 10 comprend au moins deux deuxièmes dispositifs 25. Par exemple, les deuxièmes dispositifs 25 propres, chacun, à stimuler un organe C respectif.

En variante, au moins deux deuxièmes dispositifs 25 sont configurés pour stimuler un même organe C. Par exemple, l'un des deuxièmes dispositifs 25 est implanté dans le ventricule gauche et l'autre deuxième dispositif 25 est implanté dans le ventricule droit. La stimulation du cœur du patient P est alors plus proche de la situation physiologique que lorsque le système implantable 10 comporte un seul deuxième dispositif 25. Le système implantable 10 est alors particulièrement adapté au cas du traitement de certains types d'insuffisances cardiaques.

Selon une autre variante, le deuxième dispositif 25 ne comporte pas de deuxième émetteur/récepteur 120. Dans ce mode de réalisation, l'onde acoustique émise par le transmetteur 70 et reçue par le convertisseur 125 est la seule forme de communication entre le dispositif de centralisation 20 et le deuxième dispositif 25.

Un deuxième exemple de système implantable 10 va maintenant être décrit. Les éléments identiques au premier exemple de système implantable 10 de la figure 1 ne sont pas décrits à nouveau. Seules les différences sont mises en évidence.

Le stimulateur 115 comporte une deuxième réserve d'énergie électrique. La deuxième réserve comporte, par exemple, un condensateur. La deuxième réserve est propre à recevoir le troisième courant d'alimentation C3 et à stocker au moins une partie de l'énergie électrique du troisième courant d'alimentation C3.

Le stimulateur 115 est configuré pour générer une impulsion électrique à partir de l'énergie électrique stockée dans la deuxième réserve.

Le fonctionnement du deuxième exemple va maintenant être décrit.

Les première, deuxième et troisième étapes sont identiques aux première, deuxième et troisième étapes du premier exemple.

La quatrième étape présente une durée strictement supérieure à la période temporelle. La durée est, par exemple, supérieure ou égale à une heure, en particulier supérieure ou égale à une semaine.

Par exemple, la quatrième étape débute lors de la réception du message d'activation par le deuxième dispositif 25 et s'achève suite à la réception d'un message de désactivation. Le message de désactivation est un message radiofréquence. Par exemple, le message de désactivation est généré par un dispositif extérieur au système implantable 10 sur commande d'un médecin lorsque le système implantable 10 doit être retiré du corps du patient P.

La cinquième étape présente une durée identique à la quatrième étape. Au cours de la cinquième étape, la deuxième réserve est alimentée avec le troisième courant d'alimentation C3. La deuxième réserve se charge donc progressivement en énergie électrique.

Au cours de la sixième étape, le dispositif de centralisation 20 transmet au deuxième émetteur/récepteur 120 un message de commande d'une stimulation de l'organe C par le stimulateur 115.

En réponse au message de commande, le stimulateur 115 génère une impulsion électrique.

L'impulsion électrique est conduite au cœur du patient P par l'électrode 130. Le cœur du patient se contracte alors en réponse au troisième courant électrique C3.

La sixième étape est mise en œuvre périodiquement avec la période temporelle.

La septième étape n'est pas mise en œuvre.

Dans le deuxième exemple, l'alimentation en énergie du deuxième dispositif 25 est continue. L'amplitude de l'onde utilisée est alors plus faible. Le système implantable 10 est donc compatible avec un plus grand nombre de transmetteurs 70.

Un troisième exemple de système implantable 10 va maintenant être décrit. Les éléments identiques au premier exemple de système implantable 10 de la figure 1 ne sont pas décrits à nouveau. Seules les différences sont mises en évidence.

Le deuxième dispositif 25 comporte au moins un capteur 117. Chaque capteur 117 est propre à mesurer une valeur d'un paramètre représentatif d'un phénomène physiologique du patient P. Le deuxième émetteur/récepteur 120 est alors configuré pour transmettre au dispositif de centralisation 20 les valeurs mesurées.

Le premier contrôleur 45 est configuré pour détecter au moins un phénomène physiologique se produisant chez le patient P. En particulier, le premier contrôleur 45 est configuré pour détecter le phénomène physiologique à partir des valeurs mesurées par le capteur intégré dans le deuxième dispositif 25.

Le phénomène physiologique est, par exemple, une apnée du sommeil. Par exemple, l'organe C est le nerf phrénique.

Dans ce cas, le capteur 117 est propre à détecter un mouvement du diaphragme du patient P, indicatif d'une inspiration. Lorsqu'aucune inspiration n'a été détectée pendant une durée prédéterminée, le premier contrôleur 45 détecte une apnée du sommeil.

Lorsque le phénomène physiologique est détecté, le dispositif de centralisation 20 commande en réponse la stimulation de l'organe C. En particulier, le deuxième dispositif 25 stimule électriquement l'organe C en réponse à la commande transmise par le dispositif de centralisation 20.

Par exemple, le dispositif de centralisation 20 commande la stimulation, par le deuxième dispositif 25, du nerf phrénique du patient. La stimulation du nerf phrénique déclenche alors une toux débloquant les voies aériennes supérieures du patient P.

En variante, l'organe C est le diaphragme. La stimulation déclenche alors une contraction réflexe du diaphragme qui entraîne une inspiration.

Selon une autre variante, le phénomène physiologique est un trouble du rythme cardiaque, par exemple une bradycardie ou une syncope. Dans ce cas, le ou les capteurs 117 sont propres à mesurer des valeurs de paramètres relatifs aux troubles du rythme tels qu'une activité électrique ou mécanique du cœur. Par exemple, le ou les capteurs 117 sont propres à mesurer une différence de potentiel entre deux électrodes et/ou une accélération causée par une contraction cardiaque. Dans ce cas, le deuxième dispositif 25 est propre à stimuler le cœur.

Selon une autre variante, au moins un capteur 117 est propre à mesurer un taux d'un marqueur biologique dans un fluide corporel F du patient P.

Selon un quatrième exemple, le dispositif de centralisation 20 comporte au moins un capteur 117. Par exemple, le dispositif de centralisation 20 comporte deux capteurs 117.

Chaque capteur 117 est extérieur au premier contrôleur 45 mais est propre à communiquer avec le premier contrôleur 45.

Chaque capteur 117 est configuré pour mesurer des valeurs d'un paramètre physiologique du patient P. Le paramètre physiologique est, par exemple, un paramètre de l'organe C.

Par exemple, au moins un capteur 117 est propre à mesurer des valeurs d'un paramètre du cœur.

Par exemple, un capteur 117 est propre à mesurer une valeur d'une accélération du dispositif de centralisation 20, telle qu'une accélération causée par une contraction du cœur C.

En variante ou en complément, un capteur 117, par exemple intégré au dispositif de centralisation 20, est propre à mesurer une valeur d'une différence de potentiel électrique entre deux électrodes du capteur 117. La différence de potentiel électrique est, par exemple, mesurée entre deux points de la paroi stomacale, c'est-à-dire que les deux électrodes sont en contact avec la paroi stomacale. En variante, le capteur 117 ne comporte qu'une électrode, et est propre à mesurer la différence de potentiel électrique entre l'électrode et l'ancre 15.

Un cinquième exemple de système implantable 10 va maintenant être décrit. Les éléments identiques au premier exemple de système implantable 10 ne sont pas décrits à nouveau. Seules les différences sont mises en évidence.

L'alimentation électrique 55 ne comporte pas de troisième connecteur 85 ni de réserve d'énergie électrique 90.

L'alimentation électrique 55 comporte un générateur d'énergie électrique. Il est entendu par « générateur d'énergie électrique » que le générateur d'énergie électrique n'est pas configuré pour être chargé en énergie électrique par un courant électrique.

Le générateur d'énergie électrique est propre à générer au moins un courant électrique par réaction d'au moins une espèce chimique présente dans le corps du patient P. Plus précisément, le générateur d'énergie électrique est propre à générer le premier courant d'alimentation C1 et le deuxième courant d'alimentation C2.

Par exemple, le générateur d'énergie électrique comprend deux électrodes, les électrodes baignant dans les sucs gastriques du patient P lorsque le dispositif de centralisation 20 est dans la position de fixation. En variante, les électrodes du générateur d'énergie électrique sont prévues pour baigner dans l'intestin du patient P lorsque le dispositif de centralisation 20 est dans la position de fixation.

Chaque électrode comporte au moins une enzyme. En variante, chaque électrode comporte au moins un micro-organisme. Par exemple, chaque électrode du générateur d'énergie électrique comporte un conducteur électrique recouvert de l'enzyme ou du micro-organisme, l'ensemble ainsi formé étant entouré d'une membrane. La membrane est, par exemple, configurée pour être traversée par certaines espèces chimiques naturellement présentes dans l'estomac ou l'intestin du patient P.

Lorsque les électrodes du générateur d'énergie électrique sont immergées dans les sucs gastriques ou dans le liquide intestinal, l'une des électrodes joue le rôle d'anode dans une réaction d'oxydo-réduction mettant en jeu une première espèce chimique. Simultanément, l'autre électrode joue le rôle de cathode dans une réaction d'oxydoréduction mettant en jeu une deuxième espèce chimique.

Par l'oxydation et la réduction simultanée de la première espèce chimique et de la deuxième espèce chimique, une tension électrique apparaît entre les deux conducteurs électriques. Le premier courant d'alimentation C1 et le deuxième courant d'alimentation C2 sont alors générés.

La première espèce chimique est, par exemple, le glucose. La deuxième espèce chimique est, par exemple, l'oxygène.

Le sixième exemple de système implantable 10 ne nécessite pas de charger électriquement une réserve d'énergie électrique 90 ni de faire pénétrer la réserve d'énergie électrique 90 dans le corps du patient P.

Les contraintes pour le patient P sont, là encore, réduites.

Selon un sixième exemple, le générateur d'énergie électrique est propre à générer au moins un courant électrique par conversion d'une énergie mécanique en énergie électrique. En particulier, le générateur d'énergie électrique est propre à générer au moins un courant électrique à partir des mouvements de l'estomac 30.

Dans la description ci-dessus, les fonctions du système implantable 10 ont été séparées en plusieurs exemples pour faciliter leur compréhension par le lecteur. Cependant, il est à noter que les exemples précédents peuvent être combinés pour générer de nouveaux modes de réalisation.

Par exemple, lorsque le deuxième dispositif 25 est un dispositif de stimulation de l'organe C, le dispositif de centralisation 20 est susceptible de comporter un capteur 117, la période temporelle de stimulation de l'organe C étant alors calculée par le premier contrôleur 45 à partir des valeurs mesurées par le capteur 117.

De plus, la description ci-dessus a été donnée dans le cas où l'ancre 15 et le dispositif de centralisation 20 forment deux dispositifs séparés. L'homme du métier comprendra aisément que le dispositif de centralisation 20 et l'ancre 15 sont susceptibles de former un seul dispositif, l'ancre 15 et le dispositif de centralisation 20 n'étant alors pas séparables l'un de l'autre. Par exemple, l'ancre 15 est venue de matière avec le boîtier 65 du dispositif de centralisation 20.

Selon encore un exemple, la tête 35 comporte au moins une semelle située à l'extérieur de l'estomac 30. Par exemple, la tête 35 comporte deux semelles.

Chaque semelle est configurée pour venir en appui contre la face extérieure de la paroi de l'estomac 30 et pour être reliée au dispositif implantable 20 de manière à exercer une force tendant à plaquer le dispositif implantable 20 contre la face intérieure de la paroi de l'estomac 30. Selon une variante, chaque semelle est configurée pour être placée entre le feuillet viscéral et le feuillet pariétal du péritoine et pour venir en appui contre le feuillet viscéral pour plaquer le dispositif implantable 20 contre la face intérieure de la paroi de l'estomac 30.

Chaque semelle est, par exemple, une plaque. En variante, chaque semelle comporte un treillis de fils tendus sur un cadre, en particulier un cadre souple propre à être plié et inséré dans un endoscope ou une aiguille creuse.

Le premier connecteur 40 comporte, par exemple, un ou plusieurs anneaux solidaires du boîtier 65. Chaque semelle est, par exemple, fixée au dispositif implantable 20 par un ou plusieurs fils fixés à un ou plusieurs des anneaux.

La fixation par une ou plusieurs semelles permet de répartir la pression exercée par le dispositif implantable sur une plus grande surface de la paroi de l'estomac 30 et de diminuer donc la pression ainsi exercée. En outre, ce mode de fixation ne suppose pas de générer dans la paroi stomacale un pli qui réduit le volume de l'estomac, qui est susceptible de générer des tensions dans l'ancre qui y est fixée. Puisque les forces exercées sur la paroi stomacale sont réduites, les risques d'apparition d'une réaction inflammatoire de la muqueuse gastrique sont limités.

## Revendications

1. Système implantable (10) comprenant :
- un premier dispositif (20), appelé dispositif de centralisation, propre à être implanté dans une position de fixation à l'intérieur du corps du patient (P) et
- au moins un deuxième dispositif (25) comportant une électrode (130) propre à connecter électriquement le deuxième dispositif (25) à un organe (C) du patient (P) lorsque le deuxième dispositif (25) est implanté, dans une position de stimulation, dans le corps du patient (P),
le premier dispositif (20) étant, en outre, configuré pour commander la transmission, par l'électrode (130), d'un courant électrique de stimulation de l'organe (C) par le deuxième dispositif (25),
le système implantable comprenant en outre, une ancre (15) comportant une tête (35) configurée pour ancrer l'ancre à la paroi de l'estomac (30) du patient, l'ancre (15) étant configurée pour être fixée au premier dispositif (20) et pour maintenir le premier dispositif (20) dans sa position de fixation lorsque l'ancre (15) est fixée dans l'estomac (30), le premier dispositif (20) étant accueilli dans l'estomac (30) lorsque le premier dispositif (20) est dans sa position de fixation,
le système implantable étant **caractérisé en ce que** le premier dispositif (20) est configuré pour alimenter en énergie le deuxième dispositif (25) :
- le premier dispositif (20) comporte un émetteur d'ondes, et
- le deuxième dispositif (25) comporte un stimulateur (115) propre à stimuler l'organe (C) et un convertisseur (125) propre à recevoir les ondes émises par l'émetteur et à générer en réponse un courant électrique (C3) d'alimentation du stimulateur (115),
- l'émetteur étant choisi parmi un émetteur d'ondes acoustiques et un émetteur d'ondes électromagnétiques, le convertisseur (125) étant un convertisseur (125) d'énergie acoustique en énergie électrique, propre à recevoir les ondes acoustiques émises, lorsque l'émetteur est un émetteur d'ondes acoustiques, ou un convertisseur (125) propre à recevoir les ondes électromagnétiques émises, lorsque l'émetteur est un émetteur d'ondes électromagnétiques.

2. Système implantable (10) selon la revendication 1, dans lequel l'onde acoustique est une onde ultrasonore.

3. Système implantable selon l'une quelconque des revendications 1 et 2, dans lequel le convertisseur (125) d'énergie acoustique en énergie électrique comporte des éléments piézo-électriques propres à convertir une force en tension électrique.

4. Système implantable selon la revendication 1, dans lequel le convertisseur (125) propre à recevoir les ondes électromagnétiques émises comporte une bobine propre à résonner à la fréquence de l'onde électromagnétique émise.

5. Système implantable (10) selon l'une quelconque des revendications 1 à 4, dans lequel l'électrode (130) est propre à connecter électriquement le deuxième dispositif (25) au cœur du patient (P).

6. Système implantable (10) selon la revendication 5, comportant au moins deux deuxièmes dispositifs (25).

7. Système implantable (10) selon l'une quelconque des revendications 1 à 4, dans lequel l'électrode (130) est propre à connecter électriquement le deuxième dispositif (25) à un nerf du patient (P).

8. Système implantable (10) selon la revendication 7, dans lequel l'électrode (130) est propre à connecter électriquement le deuxième dispositif (25) au nerf phrénique du patient (P).

9. Système implantable (10) selon l'une quelconque des revendications 1 à 4, dans lequel l'électrode (130) est propre à connecter électriquement le deuxième dispositif (25) au diaphragme du patient (P).

10. Système implantable (10) selon l'une quelconque des revendications 1 à 9, dans lequel le dispositif de centralisation (20) ou le deuxième dispositif (25) comporte au moins un capteur (117) propre à mesurer au moins une valeur d'un paramètre de l'organe (C) et le dispositif de centralisation (20) comprend un contrôleur (45) propre à commander la stimulation de l'organe (C), par le deuxième dispositif, en fonction de la ou les valeurs mesurées.

11. Système implantable (10) selon l'une quelconque des revendications 1 à 10, dans lequel le dispositif de centralisation (20) comprend un contrôleur (45) et une alimentation électrique (55) comportant une réserve (90) d'énergie électrique amovible et un connecteur (85) propre à accueillir la réserve d'énergie électrique (90), la réserve d'énergie électrique (90) étant propre à alimenter électriquement le contrôleur (45) lorsque la réserve d'énergie électrique (90) est connectée électriquement au connecteur (85) dans une position de connexion et de préférence étant configurée pour être avalée par le patient (P) et pour se déplacer spontanément jusqu'à la position de connexion depuis une position de déconnexion dans laquelle la réserve d'énergie électrique (90) est accueillie dans l'estomac (30) du patient (P) et est déconnectée du connecteur (85).

12. Système implantable (10) selon l'une quelconque des revendications 1 à 10, dans lequel le dispositif de centralisation (20) comprend un contrôleur (45) et une alimentation électrique (55) propre à générer un courant électrique d'alimentation du contrôleur (45) par réaction d'au moins une espèce chimique présente dans le corps du patient (P), notamment le glucose.

13. Système implantable (10) selon l'une quelconque des revendications 1 à 10, dans lequel le dispositif de centralisation (20) comprend un contrôleur (45) et une alimentation électrique (55) propre à générer un courant électrique d'alimentation du contrôleur (45) par conversion d'énergie mécanique en énergie électrique.

## Patentansprüche

1. Implantierbares System (10), umfassend:
- eine als Zentralisierungsvorrichtung bezeichnete erste Vorrichtung (20), die dazu geeignet ist, an einer Fixierungsposition im Inneren des Körpers des Patienten (P) implantiert zu werden, und
- mindestens eine zweite Vorrichtung (25), die eine Elektrode (130) umfasst, welche dazu geeignet ist, die zweite Vorrichtung (25) elektrisch mit einem Organ (C) des Patienten (P) zu verbinden, wenn die zweite Vorrichtung (25) an einer Stimulationsposition im Körper des Patienten (P) implantiert ist,
wobei die erste Vorrichtung (20) weiter dafür konfiguriert ist, die Übertragung eines elektrischen Stroms zur Stimulation des Organs (C) durch die Elektrode (130) durch die zweite Vorrichtung (25) zu steuern,
wobei das implantierbare System weiter einen Anker (15) umfasst, der einen Kopf (35) umfasst, welcher dafür konfiguriert ist, den Anker an der Wand des Magens (30) des Patienten zu verankern, wobei der Anker (15) dafür konfiguriert ist, an der ersten Vorrichtung (20) fixiert zu werden, und dafür, die erste Vorrichtung (20) an ihrer Fixierungsposition zu halten, wenn der Anker (15) im Magen (30) fixiert ist, wobei die erste Vorrichtung (20) im Magen (30) aufgenommen wird, wenn sich die erste Vorrichtung (20) an ihrer Fixierungsposition befindet,
wobei das implantierbare System **dadurch gekennzeichnet ist, dass** die erste Vorrichtung (20) dafür konfiguriert ist, die zweite Vorrichtung (25) mit Energie zu versorgen:
- die erste Vorrichtung (20) einen Wellensender umfasst, und
- die zweite Vorrichtung (25) einen Stimulator (115), der dazu geeignet ist, das Organ (C) zu stimulieren, und einen Wandler (125) umfasst, der dazu geeignet ist, die vom Sender ausgesendeten Wellen zu empfangen und in Reaktion einen elektrischen Strom (C3) zur Versorgung des Stimulators (115) zu erzeugen,
- wobei der Sender ausgewählt ist aus einem Schallwellensender und einem Elektromagnetwellensender, wobei es sich beim Wandler (125) um einen Wandler (125) von Schallenergie in elektrische Energie handelt, der dazu geeignet ist, die ausgesendeten Schallwellen zu empfangen, wenn der Sender ein Schallwellensender ist, oder um einen Wandler (125), der dazu geeignet ist, die ausgesendeten elektromagnetischen Wellen zu empfangen, wenn der Sender ein Elektromagnetwellensender ist.

2. Implantierbares System (10) nach Anspruch 1, wobei es sich bei der Schallwelle um eine Ultraschallwelle handelt.

3. Implantierbares System nach einem der Ansprüche 1 und 2, wobei der Wandler (125) von Schallenergie in elektrische Energie piezoelektrische Elemente umfasst, die dazu geeignet sind, eine Kraft in elektrische Spannung umzuwandeln.

4. Implantierbares System nach Anspruch 1, wobei der Wandler (125), der dazu geeignet ist, die ausgesendeten elektromagnetischen Wellen zu empfangen, eine Spule umfasst, die dazu geeignet ist, auf der Frequenz der ausgesendeten elektromagnetischen Welle zu schwingen.

5. Implantierbares System (10) nach einem der Ansprüche 1 bis 4, wobei die Elektrode (130) dazu geeignet ist, die zweite Vorrichtung (25) elektrisch mit dem Herzen des Patienten (P) zu verbinden.

6. Implantierbares System (10) nach Anspruch 5, das mindestens zwei zweite Vorrichtungen (25) umfasst.

7. Implantierbares System (10) nach einem der Ansprüche 1 bis 4, wobei die Elektrode (130) dazu geeignet ist, die zweite Vorrichtung (25) elektrisch mit einem Nerv des Patienten (P) zu verbinden.

8. Implantierbares System (10) nach Anspruch 7, wobei die Elektrode (130) dazu geeignet ist, die zweite Vorrichtung (25) elektrisch mit dem Zwerchfellnerv des Patienten (P) zu verbinden.

9. Implantierbares System (10) nach einem der Ansprüche 1 bis 4, wobei die Elektrode (130) dazu geeignet ist, die zweite Vorrichtung (25) elektrisch mit dem Zwerchfell des Patienten (P) zu verbinden.

10. Implantierbares System (10) nach einem der Ansprüche 1 bis 9, wobei die Zentralisierungsvorrichtung (20) oder die zweite Vorrichtung (25) mindestens einen Sensor (117) umfasst, der dazu geeignet ist, mindestens einen Wert eines Parameters des Organs (C) zu messen, und die Zentralisierungsvorrichtung (20) eine Steuerung (45) umfasst, die dazu geeignet ist, die Stimulation des Organs (C) durch die zweite Vorrichtung in Abhängigkeit von dem oder den gemessenen Werten zu steuern.

11. Implantierbares System (10) nach einem der Ansprüche 1 bis 10, wobei die Zentralisierungsvorrichtung (20) eine Steuerung (45) und eine elektrische Versorgung (55) umfasst, die eine abnehmbare elektrische Energiereserve (90) und einen Verbinder (85) umfasst, der dazu geeignet ist, die elektrische Energiereserve (90) aufzunehmen, wobei die elektrische Energiereserve (90) dazu geeignet ist, die Steuerung (45) elektrisch zu versorgen, wenn die elektrische Energiereserve (90) in einer Verbindungsposition elektrisch mit dem Verbinder (85) verbunden ist, und vorzugsweise dafür konfiguriert ist, vom Patienten (P) geschluckt zu werden, und dafür, sich spontan von einer Trennungsposition, in der die elektrische Energiereserve (90) im Magen (30) des Patienten (P) aufgenommen wird und vom Verbinder (85) getrennt ist, zur Verbindungsposition zu bewegen.

12. Implantierbares System (10) nach einem der Ansprüche 1 bis 10, wobei die Zentralisierungsvorrichtung (20) eine Steuerung (45) und eine elektrische Versorgung (55) umfasst, die dazu geeignet ist, durch Reaktion mindestens einer chemischen Spezies, die im Körper des Patienten (P) vorhanden ist, insbesondere der Glucose, einen elektrischen Strom zur Versorgung der Steuerung (45) zu erzeugen.

13. Implantierbares System (10) nach einem der Ansprüche 1 bis 10, wobei die Zentralisierungsvorrichtung (20) eine Steuerung (45) und eine elektrische Versorgung (55) umfasst, die dazu geeignet ist, durch Umwandlung von mechanischer Energie in elektrische Energie einen elektrischen Strom zur Versorgung der Steuerung (45) zu erzeugen.

## Claims

1. Implantable system (10) comprising:
- a first device (20), called a centralisation device, able to be implanted in a fixing position inside the body of the patient (P), and
- at least one second device (25) comprising an electrode (130) able to electrically connect the second device (25) to an organ (C) of the patient (P) when the second device (25) is implanted, in a stimulation position, in the body of the patient (P),
the first device (20) furthermore being configured to control the transmission, by the electrode (130), of an electric current for stimulation of the organ (C) by the second device (25),
the implantable system furthermore comprising an anchor (15) comprising a head (35) configured to anchor the anchor on the wall of the stomach (30) of the patient, the anchor(15) being configured to be fixed to the first device (20) and to hold the first device (20) in the fixing position thereof when the anchor (15) is fixed in the stomach (30), the first device (20) being accommodated in the stomach (30) when the first device (20) is in the fixing position thereof,
the implantable system being **characterised in that** the first device (20) is configured to supply energy to the second device (25):
- the first device (20) comprises a wave emitter, and
- the second device (25) comprises a stimulator (115) able to stimulate the organ (C) and a converter (125) able to receive the waves emitted by the emitter and in response to generate an electric current (C3) supplying the stimulator (115),
- the emitter being chosen from among an acoustic wave emitter and an electromagnetic wave emitter, the converter (125) being a converter (125) converting acoustic energy into electrical energy, able to receive the acoustic waves emitted, when the emitter is an acoustic wave emitter, or a converter (125) able to receive the electromagnetic waves emitted, when the emitter is an electromagnetic wave emitter.

2. Implantable system (10) according to claim 1, wherein the acoustic wave is an ultrasound wave.

3. Implantable system according to any one of claims 1 and 2, wherein the converter (125) for converting acoustic energy into electrical energy comprises piezoelectric elements able to convert a force into electrical voltage.

4. Implantable system according to claim 1, wherein the converter (125) able to receive the electromagnetic waves emitted comprises a coil able to resonate at the frequency of the electromagnetic wave emitted.

5. Implantable system (10) according to any one of claims 1 to 4, wherein the electrode (130) is able to electrically connect the second device (25) to the heart of the patient (P).

6. Implantable system (10) according to claim 5, comprising at least two second devices (25).

7. Implantable system (10) according to any one of claims 1 to 4, wherein the electrode (130) is able to electrically connect the second device (25) to a nerve of the patient (P).

8. Implantable system (10) according to claim 7, wherein the electrode (130) is able to electrically connect the second device (25) to the phrenic nerve of the patient (P).

9. Implantable system (10) according to any one of claims 1 to 4, wherein the electrode (130) is able to electrically connect the second device (25) to the diaphragm of the patient (P).

10. Implantable system (10) according to any one of claims 1 to 9, wherein the centralisation device (20) or the second device (25) comprises at least one sensor (117) able to measure at least one value of a parameter of the organ (C) and the centralisation device (20) comprises a controller (45) able to control the stimulation of the organ (C), by the second device, according to the value or values measured.

11. Implantable system (10) according to any one of claims 1 to 10, wherein the centralisation device (20) comprises a controller (45) and an electrical supply (55) comprising a removable electrical-energy store (90) and a connector (85) able to accommodate the electrical-energy store (90), the electrical-energy store (90) being able to electrically supply the controller (45) when the electrical-energy store (90) is electrically connected to the connector (85) in a connection position and preferably being configured so as to be swallowed by the patient (P) and to move spontaneously to the connection position from a disconnection position wherein the electrical-energy store (90) is accommodated in the stomach (30) of the patient (P) and is disconnected from the connector (85).

12. Implantable system (10) according to any one of claims 1 to 10, wherein the centralisation device (20) comprises a controller (45) and an electrical supply (55) able to generate an electric current supplying the controller (45) by the reaction of at least one chemical species present in the body of the patient (P), in particular glucose.

13. Implantable system (10) according to any one of claims 1 to 10, wherein the centralisation device (20) comprises a controller (45) and an electrical supply (55) able to generate an electric current supplying the controller (45) by the conversion of mechanical energy into electrical energy.
